(19) Europäisches Patentamt
European Patent Office
Office européen des brevets

(11) **EP 4 394 441 A1**

(12) **EUROPEAN PATENT APPLICATION**

(43) Date of publication:
**03.07.2024 Bulletin 2024/27**

(21) Application number: **22315360.2**

(22) Date of filing: **29.12.2022**

(51) International Patent Classification (IPC):
**G01S 7/52** (2006.01)    **G01S 15/89** (2006.01)
**A61B 8/08** (2006.01)    **G10K 11/34** (2006.01)

(52) Cooperative Patent Classification (CPC):
**G01S 15/8915; G01S 7/5202; G01S 7/52026;
G01S 7/52046; G01S 15/8997; G10K 11/346;**
G01S 15/8925

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **SUPERSONIC IMAGINE
13290 Aix en Provence (FR)**

(72) Inventors:
• **Frappart, Thomas
13090 Aix-en-Provence (FR)**
• **Lambert, William
13008 Marseille (FR)**

(74) Representative: **Paustian & Partner Patentanwälte
mbB
Oberanger 32
80331 München (DE)**

(54)    **A BEAMFORMING METHOD AND SYSTEM**

(57)    The invention relates to a beamforming method (100), comprising:
reconstructing a synthetic transmit beam (STB) based on adjacent focalized beams (b1, b2) transmitted into a medium (M), wherein
the adjacent focalized beams (b1, b2) have different focal depths (FD1, FD2).

Fig. 2B

**Description**

BACKGROUND

It is known to use transducer for communication, imaging or scanning purposes, for example in the field of medical imaging, radar, sonar, seismology, wireless communications, radio astronomy, acoustics and biomedicine. One example comprises ultrasound imaging.

One aim of ultrasound imaging is to create an ultrasound image of a medium under investigation. This ultrasound image, also called B-mode image is an estimation of the medium reflectivity. In a conventional ultrasound imaging method, an ultrasound transducer device (also referred to as an ultrasound probe) with a set of ultrasound transducer elements may be used. In the method, one or multiple transducers are used to transmit one or successively several ultrasound beams into a medium, corresponding to a transmission operation. For each transmitted beam, the backscattered echoes generated by the medium are received by the same set or another set of transducer elements. In particular, each of the transducer elements converts a received echo signal into for example an electrical signal. The signal may further be processed by the ultrasound system or by any associated (optionally dedicated) system. For example, they may be amplified, filtered, digitalized and/or a signal conditioning operation may be carried out. The transducer elements may be arranged as a transducer line, transducer array, matrix transducer, three-dimensional (3D) transducer, or any other configuration.

Conventionally, said signals are then transmitted to an image processing system. The received signals may be processed to generate image data of the scanned medium, for example using a beamforming method, more in particular a delay and sum (DAS) beamforming method. Generally, beamforming may be understood as a signal processing technique conventionally used in sensor arrays for directional signal transmission and/or reception. This process is used to generate beamformed data. In other words: beamforming may be understood as a signal processing technique that is achieved by combining elements in an antenna array (for example an ultrasound transducer) in such a way that the combined signals form constructive interferences.

SUMMARY OF THE DISCLOSURE

The present disclosure relates to a beamforming method. The method comprises reconstructing a synthetic transmit beam based on adjacent focalized beams transmitted into a medium. Furthermore, the adjacent focalized beams have different focal depths.

A synthetic transmit beam may be reconstructed by any. signal processing technique. In this context, the term "reconstructing" may be also understood as "synthetizing" or "beamforming". The term "synthetic" and "reconstructing" may refer to using the information generated by scatterers located within areas that are overlapping between the adjacent focalized beams. For example, "reconstructing" may refer to coherently summing information, one can retrospectively re-create virtual transmit focus beams at each spatial region which is associated with or represented a respective pixel of a constructed image. A synthetic transmit beam may be reconstructed in any overlapping area of the adjacent focalized beams.

The focalized beams are transmitted into a medium and may therefore be regarded as physical beams (in contrast to for example the synthetic transmit beam). The focalized beams may be transmitted using a plurality of transducer elements. As an example, the focalized beams may be signal beams. For instance, the plurality of transducer elements may be arranged in a single plane. However, it is also possible that the transducer elements are arranged in a matrix form (i.e. to permit a three-dimensional imaging, as described below). In the following disclosure it is in particular referred to examples of using transducer elements arranged in a single plane to generate two-dimensional (2D) images of the medium. This exemplary disclosure may be extended to examples using transducer elements arranged in a matrix form to generate three-dimensional (3D) images of the medium.

A transmitted focalized beam may have any shape. For example, a focalized beam may essentially have a double cone shape. A focalized beam may have essentially the profile shape of a Gaussian beam, such as those beam profile shapes used in laser technology or optics. In particular, a focalized beam may have a wide extension in radial direction at a starting point, for example at the plurality of transducer elements, a narrow extension in radial direction at the focal depth (also called waist) and a wide extension in radial direction in a far field. A starting point may be located at the plurality of transducer elements. A far field may be or may comprise a point or area more distanced from the transducer elements than the focal point of the transmitted focalized beam. For example, the far field may correspond to a distance, which is larger than the Fraunhofer distance.

As described in more detail below, a transmitted focalized beam may be generated electronically, i.e. by respectively delaying single pulses generated by adjacent transducer elements, in order to focalize an overall wave to a predefined focal area.

Adjacent focalized beams may be, for example, two focalized beams that are transmitted by the plurality of transducer elements, wherein each of the focalized beams is transmitted with different settings for a synthetic aperture. In the synthetic aperture approach, the plurality of transducer elements may be fired element by element, and the complete set of impulse responses between each transmit and receive element is beamformed and recorded. The setting of a synthetic aperture may be dependent on the number of firing elements, the sequence of firing and the delay between firing elements.

A focalized beam may have a focal region.

Focalized beams with different focal depths may be, for example, one focalized beam with the focal region closer to the plurality of transducer elements and another focalized beam with the focal region more distanced from the plurality of transducer elements.

In general, the method according to the present disclosure may also be applied to any technical field, such as ultrasound examination. In particular, any technical field is possible which uses a plurality of transducer elements to acquire data/signals of an examined medium or environment and/or which may optionally use a beamforming technique based on the collected data/signals. Examples comprise methods using a radar system, sonar system, seismology system, wireless communications system, radio astronomy system, acoustics system, Non-Destructive Testing (NDT) system and biomedicine system. The method may be in particular suitable for obtaining information about a medium.

A medium is, for instance, a living body and in particular human or animal bodies, or can be any other biological or physic-chemical medium (e.g. in vitro medium). Accordingly, a medium may comprise living tissues. For example, the medium may comprise tissues such as fat, muscles, bones, and blood vessels, each one having various physical properties. A medium may also be non-living material, for example gravel, lava, or weldings.

The medium may comprise variations in its physical properties. In further examples, the tissue may comprise an area suffering from a disfunction and/or an illness (e.g. cancerous cells, muscular tearing, ...), or any other singular area, having various physical properties in comparison to other area of the medium.

One advantage of a synthetic transmit beam is a high speed. Furthermore, the method is not only faster but also better in focalizing on one specific point as the used number of transducers may be greater, i.e. larger. Therefore, a stronger focalization may be applied in areas without an overlap. Another advantage due to different focal depths is that the synthetic transmit beams even more differ and a better coherent summation may be achieved.

In one aspect, the focal depths may be defined as a function of a depth of a region of interest located in the observed medium.

The region of interest may be a predefined region of interest, for example, selected by a user and/or by an algorithm. The region of interest may be a predefined region of interest set as a default value. The region of interest may be a 2D (two dimensional) or 3D (three dimensional) spatial region. The region of interest may have any shape or dimensional extension. For example, the region of interest may have the shape of a circle, a rectangle or of a cube.

In general, it may be said that the synthetic transmit beam focalizes at a synthetic focal depth (what is actually implied by synthetic transmit beam, i.e. the STB). The synthetic focal depth may be in the region of interest. More in particular, the synthetic transmit beam may focalize in a (for example predefined) spatial region within the medium, in particular within the region of interest. A spatial region may be associated, for example, with a pixel or a voxel in image data, as described in more details below.

Accordingly, for each spatial region, a synthetic transmit beam may be reconstructed. In other words, acquired signal data may be synthetized for the (or for each) spatial region, as if a focalized beam was transmitted to focalize in said spatial region.

Accordingly, synthetic transmit beam may be reconstructed for a plurality of different spatial regions. These spatial regions may be in particular an area where the transmitted beams overlap (i.e. where signal data derived from both transmitted beams exist).

Due to the reconstruction of a synthetic transmit beam, a spatial region of the medium associated with the synthetic transmit beam (i.e. in which the synthetic transmit beam focalizes) may be reconstructed, using signal data associated with different adjacent focalized beams transmitted into the medium.

In one aspect, the focal depths may be defined to be within or beyond an extension of the region of interest in the depth direction.

Both alternatives (within or beyond an extension of the region of interest) are possible.

In general, both alternatives have advantages due to the following tradeoff: On the one hand side, the focal depths should be close to the central depth level of the region of interest, to allow a precise imaging of the region of interest, and thus close to each other. On the other hand side, the beams and thus their respective focal depths should be sufficiently different, in order to obtain more information from the medium by destructive interference of the signals related to the beams.

In one aspect, a first one of the focal depths may be above a central depth level of the region of interest and wherein a second one of the focal depths may be below the central depth level of the region of interest.

In a further example, the distance between the focal depths may be determined as a function of the depths of field of the respective adjacent focalized beams. Said distance may for example correspond to the vertical extension of one or both depths of field of the adjacent focalized beams.

Accordingly, in one example, the distance of the focal depths, FD1 and FD2, of the first and second focalized beams (b1, b2) may be calculated according to the following equation:

$$FD2 - FD1 \sim= Dz1 + Dz2 \qquad (eq. 1)$$

Where Dz1 and Dz2 may be the depth of field associated to the focalized beams (b1, b2) with the focal depths FD1 and FD2 respectively. The depth of field Dz1 (e.g. associated with the first focalized beam b1) indicates the depth range over which the beam is constrained along the scanning line. In other words, it defines whether the beam is more or less focalized. Dz1 may be approximately determined to be for example, cf. eq. 2: .

$$Dz1 = 7* lambda (FD1/D1)^2, \qquad (eq. 2)$$

wherein
D1 is the aperture of the first focalized beam (b1) at the transducer plane. It corresponds to the active length defined by the active transducers (for example defined by the number of used transducer elements and the piezo-pitch the distance between two transducers elements). Dz2 may for example be determined correspondingly, as a function of an aperture D2 of the second focalized beam (b2).

Any kind of relation between the first and the second one of the focal depths is possible. For, example, the relation between the first and the second one of the focal depths may be greater than 1.2 (cf. eq. 3a: FD1 > 1.2 * FD2), more preferably be greater than 1.5 (cf. eq. 3b: FD1 > 1.5 * FD2), still more preferably be greater than 1.8 (cf. eq. 3c: FD1 > 1.8 * FD2).

In one aspect, the first focal depth and the second focal depth may have the same distance from the central depth level of the region of interest.

Both alternatives of the previous aspects (within or beyond an extension of the region of interest) are possible, in particular in combination with having the same distance to the central depth level of the region of interest.

In one aspect, the transmitted beams may have different apertures. Furthermore, an aperture of a transmitted beam may be determined as a function of its focal depth.

In order to change a focal depth of a transmitted beam, at least one of the piezoPitch and the number of used transducer elements may be changed. In case the piezoPitch is kept constant between the adjacent focalized beams, the focal points of the beams may have the same intensity. In case the number of used transducer elements is kept constant, the same energy may be. emitted into the medium by the adjacent focalized beams.

Accordingly, the apertures of the transmitted beam may be varied as a function of the variation of the focal depths, i.e. the apertures set by the transducer device for generating the beams. For example, when the focus is less profound (i.e. lower depth), a smaller aperture may be used, i.e. less transducer elements of the transducer device and/or a varied piezoPitch may be used to generate the beam.

Moreover, also in an acquisition phase (i.e. acquisition method), the aperture may be determined as a function of the focal depth of the respective transmitted beam. The acquisition phase may comprise successively transmitting adjacent focalized beams having different focal depths into a medium. Furthermore, the acquisition phase may comprise acquiring a set signal data of the medium in response to each transmitted beam. The acquisition phase may also comprise processing the sets of signal data in a beamforming method according to any example of the present disclosure. However, this processing operation may also be part of a separate processing phase (i.e. separate to the acquisition phase).

In one aspect, more than two adjacent focalized beams may be transmitted into the medium having two or more different focal depths.

Having two or more different focal depths may increase the overlap and/or the coherent summation.

In one aspect, the different focal depths of the more than two adjacent focalized beams may be alternating.

In other words, the (more than two) beams may have alternating first and second (and optionally more) focal depths. Also, the focal depths may alternate across the scanning lines of the respective beams.

It is also possible that more than two focal depths are used, which may be selected as a function of the depth of region of interest and/or be within the depth extension of the region of interest according to any example of the present disclosure.

In one aspect, the focal depths may be selected such that the adjacent focalized beams overlap in the region of interest in the medium. It may also be possible that an overlap between the adjacent focalized beams in a region of interest of the medium is increased.

In one aspect, the adjacent focalized beams may be offset to each other. Furthermore, the adjacent focalized beams may be arranged along adjacent scanning lines.

The offset to each other may be, for example, in a direction perpendicular to a depth direction and/or a primary axis of the beams. The scanning lines may be, for example, parallel to each other.

Accordingly, the adjacent focalized beams may be adjacent in a direction perpendicular to the depth direction. In other words, the adjacent focalized beams may be adjacent in a lateral dimension of the medium. The lateral dimension may be hence any dimension (for example x,y) except the depth dimension (z).

In one aspect, the adjacent focalized beams may be transmitted by the plurality of transducer elements into the medium, wherein the scanning lines may be defined by the arrangement of the transducer elements. For example, the scanning lines may comprise to the primary emission and/or reception directions of the transducer elements. This direction may also be referred to as a depth direction (z) of the medium.

The adjacent focalized beams may be, for example, provided by a transducer device or a probe comprising an array of transducer elements.

In one aspect, the adjacent focalized beams may be based on ultrasound waves transmitted into the medium. Furthermore, the adjacent focalized beams may correspond to physical areas of the medium passed through by ultrasound waves.

In other words, the adjacent focalized beams may correspond to physical areas of the medium insonified by ultrasound waves.

In one aspect, reconstructing the synthetic transmit beam may comprise reconstructing the synthetic transmit beam based on signal data of the medium associated with the adjacent focalized beams transmitted into the medium.

The signal data may in particular be data of echo signals, i.e. they may be acquired as a function of or in response to transmitted beams according to any examples of the present disclosure.

In one aspect, the signal data comprise at least one of in-phase and quadrature phase, IQ, data wherein a phase of the IQ data may be adjusted as a function of a synthetic focal depth of the synthetic transmit beam, in relation to the focal depths of the adjacent focalized beams. More generally, the signal data may be raw data, i.e. data of backscattered echoes generated by the physical focalized beam.

Accordingly, the signal data may be processed for providing (or obtaining) complex IQ data sets. This may mean that each IQ data set is respectively associated to a synthetic transmit beam (and thus for example to an image pixel, as explained above). For example, the IQ data sets may be demodulated IQ ultrasound data sets and/or IQ pre-beamformed data sets. More generally an IQ data set may comprise IQ data. For example, an IQ data set may comprise at least one pair of an in-phase value and a quadrature phase value. Accordingly, the IQ data may be demodulated IQ ultrasound data.

The IQ data may have or may be associated with a phase.

In the present invention, each pixel may be reconstructed based on back scattered echoes generated by focused transmit beams that may have different focal depth. To do so, the beamforming process needs to be slightly. For a converging beam, a phase shift called Gouy phase shift appears at the focal depth due to the transformation from a converging wave to a diverging wave. For example, the transmitted beam may be based on a transmitted cylindrical wave (for example using a transducer device with a linear array of transducer elements). In such a case the phase shift of the IQ data of the order of pi/2 may occur. For conventional RTB where all the transmitted beams have the same focal depth, each reconstructed pixels are based on the combination of either a set of converging waves (for $z < z_{in}$, with $z_{in}$ the focal depth) or a set of diverging waves (for $z > z_{in}$). In the present disclosure, some pixels may be reconstructed based on the combination of both converging and diverging waves. This leads to an additional phase shift term in the beamforming process described below.

A more specific example of the beamforming method according to the present disclosure is described in the following in context of the following equations (4) to (10).

Generally, a transducer device may emit $N_{in}$ converging beams that focus at location $\mathbf{r}_{in} = \{z_{in}, x_{in}\}$ ($\mathbf{z}_{in}$ may for example correspond to the focal depth FD1 and/or FD2 mentioned above). The backscattered echoes maybe measured by $N_{out}$ transducers located at location $\mathbf{u}_{out}$. $IQ_r(\mathbf{r}_{in}, \mathbf{u}_{out}, t)$ may be denoted as the complex raw IQ data that corresponds to the backscattered echoes modulated at frequency $f_{demod}$, generated by the converging beam that focuses at $r_{in}$ and measured at time of flight t by the transducer located at $u_{out}$.

The IQ-beamformed data $IQ_b(\mathbf{r})$ at point $\mathbf{r} = \{z,x\}$ may be expressed as:

$$IQ_b(\mathbf{r}) = \sum_{\mathbf{r}_{in}} \sum_{\mathbf{u}_{out}} W(\mathbf{r}_{in}, \mathbf{u}_{out}, \mathbf{r}) \times IQ_r\big(\mathbf{r}_{in}, \mathbf{u}_{out}, TOF(\mathbf{r}_{in}, \mathbf{u}_{out}, \mathbf{r})\big)$$
$$\times e^{j\,2\pi\,f_{demod}\,TOF(\mathbf{r}_{in}, \mathbf{u}_{out}, \mathbf{r})} \qquad (eq.\ 4)$$

wherein parameters of eq. (4) are explained above and in the further explanations below.

Note that eq. (4) could be adapted for raw rf data instead of modulated IQ one.

In eq. (4) $TOF(\mathbf{r}_{in}, \mathbf{u}_{out}, \mathbf{r})$ may be the round-trip time of flight of the echoes to travel from the transducer device to the point $\mathbf{r}$, and to go back to the receive transducer $\mathbf{u}_{out}$ cf. eq. (5):

$$TOF(\mathbf{r}_{in}, \mathbf{u}_{out}, \mathbf{r}) = t_{in}(\mathbf{r}_{in}, \mathbf{r}) + t_{out}(\mathbf{u}_{out}, \mathbf{r}) \qquad (eq.\ 5)$$

To determine $t_{in}$ and $t_{out}$, an assumption on the medium speed of sound is require. For sake of simplicity and processing time, the medium is generally assumed as homogeneous with a constant speed of sound c. In this context, $t_{in}$ may for example be determined by, cf. eq. (6):

$$t_{in} = \frac{z_{in}}{c} + sign(z - z_{in}) \times \frac{|\mathbf{r}_{in} - \mathbf{r}|^2}{c} \qquad (eq.\ 6),$$

and $t_{out}$ may for example be determined by, cf. eq. (7):

$$t_{out} = \frac{|\mathbf{r} - \mathbf{u}_{out}|^2}{c} \qquad (eq.\ 7).$$

Moreover, $W(\mathbf{r}_{in}, \mathbf{u}_{out}, \mathbf{r})$ may be a weight that may combine in one example multiple uses, as defined in exemplary eq. (8):

$$W(\mathbf{r}_{in}, \mathbf{u}_{out}, \mathbf{r}) = W^{in}_{apod} \times W_{phase} \times W^{out}_{apod} \qquad (eq.\ 8)$$

In this example, $W^{in}_{apod}(\mathbf{r}, \mathbf{r}_{in})$ may select and potentially apply a weight on the different beams that may be set to reconstruct the beamformed IQ data at point r, cf. for instance eq. (9) :

$$W^{in}_{apod}(\mathbf{r}, \mathbf{r}_{in}) = \frac{1}{N_{in}(\mathbf{r})} \times mask(\mathbf{r}, \mathbf{r}_{in}) \qquad (eq.\ 9),$$

where *mask* may be a predefined function configured to define whether the beamformed point **r** is within the area insonified by the transmitted beam that focused at $\mathbf{r}_{in}$. This function can be determined based on the geometry or by mean of more advance simulation of the transmitted beam. Note that this mask may be more complex to embed a spatial window that may depends on the amount of energy received by the scatterers located at point r for the incident beam that focuses at $\mathbf{r}_{in}$ $W^{out}_{apod}(\mathbf{r}, \mathbf{u}_{out})$ may correspond to $W^{in}_{apod}(\mathbf{r}, \mathbf{r}_{in})$ but for the output. For instance, a received spatial window apodisation is commonly used based on the difference of horizontal coordinate of the receive transducer $\mathbf{u}_{out}$ compared to *x* the lateral coordinate of the beamformed pixel. Generally, a constant fNumber *fN = z/A* may be defined that defines the aperture size A (i.e. which receive channel are used to beamformed data at depth z).

$W_{phase}$ may correspond to the additional term used to compensate the Gouy phase shift, for instance eq. (10):

$$W_{phase}(\mathbf{r}_{in}, \mathbf{r}) = \begin{array}{l} \exp(i\pi)\,, \text{if } (z_{in} > z) \\ 0\,, \text{else} \end{array} \qquad (\text{eq. } 10)$$

It may also be possible to provide a smoothened transition of the phase shift (for example from 0 to pi/2) at the threshold $z_{in}$.

In one aspect, the method may comprise determining a physical characteristic of a spatial region in the medium as a function of the reconstructed synthetic transmit beam. It may also be possible that the synthetic transmit beam is focalized in the spatial region.

A physical characteristic may be for example image data or a reflectivity of the medium. A spatial region may correspond, for example, to a pixel or a voxel in image data.

In one aspect, the present disclosure may relate to a data acquisition method. The data acquisition method may comprise successively transmitting adjacent focalized beams having different focal depths into a medium. Furthermore, the data acquisition method may comprise acquiring a set signal data of the medium in response to each transmitted beam. The data acquisition method may also comprise processing the sets of signal data in a beamforming method according to any example of the present disclosure.

A data acquisition method may be, for example, a medical data acquisition method. A data acquisition method may comprise a reception of echo signals from the medium, and optionally a pre-processing operation, in order to obtain (digital) data, for example channel data, i.e. RF (raw) data.

Signal data may be, for example, medical data. The obtained medical data may also be referred to as raw data. For example, medical data may be related to a living being observed in order to better understand its health (e.g. sportsman) or to identify possible pathologies. Medical data may be obtained in a medical imaging method. Examples of medical imaging methods may be ultrasound imaging, tomography and/or three-dimensional (3D) imaging, X-ray imaging, mammography imaging, and magnetic resonance imaging (MRI).

Accordingly, a first set of signal data may be acquired in response to a first transmitted beam, a second set of signal data may be acquired in response to a second transmitted beam, and this may be replicated several times, i.e. continued respectively.

A synthetic transmit beam may be reconstructed based on a plurality of sets of signal data associated with a respective plurality of transmitted adjacent focalized beams (for example two sets associated with two transmitted beam).

In one aspect, the present disclosure may relate to a medical imaging method. The medical imaging method may comprise a data acquisition method according to any example of the present disclosure. Furthermore, the medical imaging method may comprise compiling image data based on the processed data.

The medical imaging method may hence comprise: A medical data acquisition method to obtain raw data, and a medical data processing method to process the raw data, in order to obtain image data. Those image data may then be displayed on the same device, optionally in real time, or later or simultaneously, on a dedicated device and/or on another display device (such as a screen of a smart phone or tablet).

In one aspect, the present disclosure may relate to a computer program comprising computer-readable instructions which when executed by a data processing unit cause the data processing unit to carry out processing operations of the method according to any examples of the present disclosure. In case the method operations may comprise any (physical) aspects which go beyond a mere data processing (for example an ultrasound wave emission), the computer program may further comprise computer-readable instructions which when executed by a data processing system cause any external elements of a system (for example an ultrasound transducer device) to carry out these operations.

The computer program may also be configured to operate one or several probes or any other external device associated with the processing system.

The present disclosure may also relate to a beamforming system. The system may comprise a processing unit configured to reconstruct a synthetic transmit beam based on adjacent focalized beams transmitted into a medium, wherein the adjacent focalized beams have different focal depths.

The beamforming system may furthermore be configured to perform any of the method features or operations described above.

The beamforming system may for example comprise or be connectable to a transducer device which acquires the ultrasound spatio-temporal signal data. It is further possible that the system receives the ultrasound spatio-temporal signal data from an external data system, for example a data storage.

External data systems may comprise program code or implementations of artificial intelligence (AI), machine learning model (ML), or machine learning algorithm. AI or machine learning models may be trained using training data, such as the spatial-temporal signal data. Furthermore, it may be possible that AI or ML are used to preprocess or postprocess any data according to examples of the present disclosure.

For example, the AI or ML may comprise supervised learning algorithms, unsupervised learning algorithms, semi-supervised learning algorithms, artificial neural network (ANN), support vector machine, a random forest model, or a gradient boosting model.

Generally, in the present disclosure a pulse may correspond to an acoustic and/or electrical signal emitted by a transducer element. The pulse may for example be defined by at least one of: the pulse duration, the frequency of the resulting wave, the number of cycles at the given frequency, the polarity of the pulse, etc. A wave may correspond to the wavefront generated by one or several transducer elements (i.e. by respectively emitted pulses). The wave may be controlled by means of emission delay between the different used transducer elements. Examples comprise a plane wave, a focused wave and a divergent wave. A beam may correspond to the physical area insonified by the wave (for example in the medium). Hence, the beam may be related to the wave but may have less or no temporal notion. For example, it may be referred to a beam when the depth of field of a focused beam is of interest.

It is intended that combinations of the above-described elements and those within the specification may be made, except where otherwise contradictory.

It is to be understood that both the foregoing general description and the following detailed description are exemplary and explanatory only, are provided for illustration purposes and are not restrictive of the disclosure, as claimed.

The accompanying drawings, which are incorporated in and constitute a part of this specification, illustrate examples of the disclosure and together with the description, and serve to support and illustrate the principles thereof.

## BRIEF DESCRIPTION OF THE DRAWINGS

Fig. 1 shows two adjacent focalized beams b1 and b2 with the same focal depths FD1 according to conventional techniques.

Fig. 2A shows a flowchart of a beamforming method 100 comprising reconstructing a synthetic transmit beam, STB, based on adjacent focalized beams transmitted into a medium according to examples of the present disclosure.

Fig. 2B shows two adjacent focalized beams b1 and b2 with different focal depths FD1 and FD2 according to the present disclosure.

Fig. 3A shows the focal depths FD1 and FD2 within an extension of the region of interest ROI in the depth direction according to examples of the present disclosure.

Fig. 3B shows the focal depths FD1 and FD2 beyond an extension of the region of interest in the depth direction according to examples of the present disclosure.

Fig. 3C shows a first one of the focal depths above a central depth level CDL of the region of interest ROI and wherein a second one of the focal depths is below the central depth level CDL of the region of interest ROI according to examples of the present disclosure.

Fig. 3D shows the first focal depth FD1 and the second focal depth FD2 having the same distance from the central depth level CDL of the region of interest ROI according to examples of the present disclosure.

Fig. 4 shows the adjacent focalized beams b1 and b2 are offset OFS to each other and the adjacent focalized beams b1 and b2 are transmitted by a plurality of transducer elements 22 into the medium according to examples of the present disclosure.

Fig. 5 more than two adjacent focalized beams b1, b2, b3, b4, b5, b6, b7, and b8 are transmitted into the medium having two different focal depths according to examples of the present disclosure.

Fig. 6A shows a schematic drawing of an ultrasound system 100 according to examples of the present disclosure.

Fig. 6B shows a schematic drawing of an ultrasound imaging system 10 according to examples of the present disclosure.

## DESCRIPTION OF THE DRAWINGS

Reference will now be made in detail to examples of the disclosure, examples of which are illustrated in the accompanying drawings. Wherever possible, the same reference numbers will be used throughout the drawings to refer to the same or like parts.

In general, conventional medical imaging focuses on examining a medium by transmitting and receiving one mode of signal. However, combining different modes may also be possible, for example to improve medical imaging data and help to get faster and more accurate examination results.

Conventional Bmode (brightness mode) imaging generally uses converging . (or focused) transmitted beams. They are defined by parameters such as their focal depth, aperture (set of transmitted transducers) and transmitted angle. Each beam results from the combination of pulses generated by each transmitted transducers elements and shaped by means of appropriate delays in transmission to focalized at the desired focal depth. The pulses are defined by parameters such as the central transmitted frequency, the pulse length, the duty cycle. Each beam is centered along an associated scanning line. The ensemble of scanning line is defined to pave the imaging plane (for 1D probes) or imaging volume (for 2D probes). There is a tradeoff between the distance between two scanning lines (i.e., the transmit line pitch) and the imaging framerate (proportional to the total number of transmitted beams used to reconstruct a Bmode image). Therefore, the transmit line pitch is usually set to a greater value than the ideal case. For example, the transmit line pitch may be set equals lambda/2 according to the spatial Nyquist criterion, wherein lambda is the wavelength of the waves used to generate the beams.

As stated before, for each focused transmitted beam centered along an associated scanning line, back-scattered echoes are generated by the medium scatterers located within the insonified area. For focused beam, this area looks like a cone. The backscattered echoes are measured by a set of receive transducers and used to estimate the medium reflectivity along the scanning line by means of a beamforming process. One of the most widely used is the delay-and-sum (DAS) beamforming. For each pixel, which is associated to a reconstructed area, this beamforming consists in coherently summing the echoes measured by the different receive transducers element and generated by scatterers located at these reconstructed areas. Theses echoes are selected based on an assumption of the round-trip travel distance for the incident beam to reach the selected area and for the back scattered echoes to back propagates toward each receive transducer.

Due to the trade-off between the frame rate and transmit line pitch, that prevent the use of optimal transmit line pitch, multiple lines are reconstructed based on a single transmitted beam. The ultrasound Bmode image is then obtained by concatenating all the reconstructed lines associated to each transmitted beam. Note that to avoid line artefact, the same focal depth is used for all the beams used to reconstruct the entire image.

As described in for example US20220287685A1, retrospective transmit beamforming (RTB), which may also be referred to as retrospective transmit focusing, synthetic transmit beamforming (STB) or true confocal imaging, is a beamforming technique that improves the image reconstruction. The idea lies in the fact that adjacent beams centered on adjacent scanning lines are overlapping one the other. Therefore, backscattered echoes generated by a given scatterer over successive adjacent beams can be combined to beamform a given pixel. In the case of DAS beamforming those echoes are coherently summed. If all the wave propagation are linear steps and if the medium has not moved during the successive insonification step, then a beamformed pixel reconstructed via this advance beamforming process (that is based on multiple insonifications) is equivalent to a conventional DAS beamforming process associated with a focused beam exactly been designed to focused on the desired target. Hence the name of retrospective transmit focusing where a transmit focusing is numerically achieved for each pixel of the reconstructed image, leading to a confocal imaging (a transmit and receive beamforming at each pixel).

Compared to conventional image reconstruction process, the RTB process improves the image quality. The lateral resolution outside the depth of field of the physical transmitted beams is improved. The contrast and signal to noise ratio are improved by using the information contained within the insonified areas that are overlapping between adjacent focalized beams. This information is simply not used in conventional beamforming; By wisely coherently summing this information, one can retrospectively re-create virtual transmit focus beams at each pixel. STB offers a new tradeoff between lateral sampling (i.e. transmit line pitch) and imaging framerate.

WO2017093778 (A1) discloses a beamforming method using synthetic beamforming for producing an image of a region inside a medium, having a reduced speckle noise.

Furthermore, beamforming methods using synthetic beamforming are known from the following scientific publications:

Moo-Ho Bae and Mok-Kun Jeong, "A study of synthetic-aperture imaging with virtual source elements in B-mode ultrasound imaging systems," in IEEE Transactions on Ultrasonics, Ferroelectrics, and Frequency Control, vol. 47, no. 6, pp. 1510-1519, Nov. 2000, doi: 10.1109/58.883540.
T. Hergum, T. Bjastad, K. Kristoffersen and H. Torp, "Parallel beamforming using synthetic transmit beams," in IEEE Transactions on Ultrasonics, Ferroelectrics, and Frequency Control, vol. 54, no. 2, pp. 271-280, February 2007, doi: 10.1109/TUFFC.2007.241.

The system and method described herein are related to technologies for beamforming, in particular in the context of medical imaging. The method is in particular suitable for obtaining information, such as a physical characteristic, about a medium. The method may be used in a device such as for instance an ultrasound system.

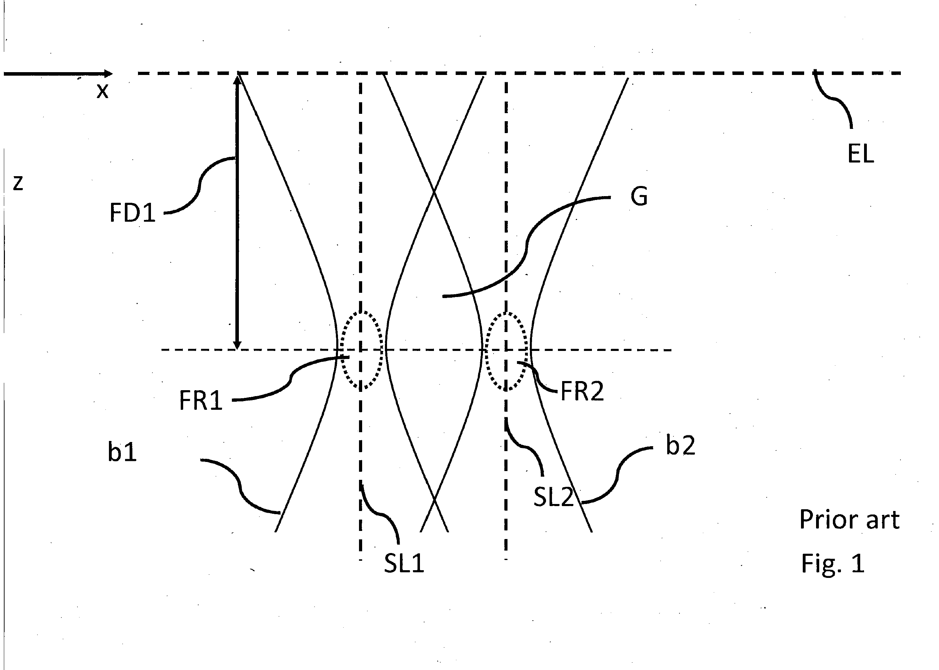

Fig. 1 shows two adjacent focalized beams b1 and b2 with the same focal depth FD1 according to conventional techniques. The adjacent focalized beams b1 and b2 are transmitted starting from an emission line EL and along a z direction into a medium. Furthermore, the adjacent focalized beams b1, b2 may be arranged along adjacent scanning lines SL1, SL2. The scanning lines SL1, SL2 may be, for example, parallel to each other. Each of the adjacent focalized beams b1 and b2 has the profile shape of a Gaussian beam, such as for example those beam profile shapes used in laser technology or optics. In particular, each of the adjacent focalized beams b1 and b2 has a wide extension in x direction at a starting point (z=0), i.e. the plurality of transducer elements, a narrow extension in x direction at the focal depth FD1 (also called "waist") and a wide extension in x direction in a far field (z>>0). STB

Each of focalized beams b1 and b2 have a respective focal region FR1 and FR2.

Due to the same focal depth FD1 and the shape of the adjacent focalized beams b1 and b2, a non-overlapping area G (also called "gap") may occur (schematically shown in fig. 1). A synthetic transmit beam, STB, may be reconstructed, for example at any area between FR1 and FR2 (not shown in fig. 1.).

One problem of this method is that there is a discontinuity in the reconstructed delay laws at the focal depth FD1. Using a spherical model introduces a singularity at the focal depth FD1 where the virtual source is located, and this discontinuity creates an artifact on the reconstructed images. Additionally, the focalized beams are the narrowest between their focal depths FD1 and FD2, such that the overlapping information between adjacent focalized beams b1 and b2 in this depth is further reduced. In some examples, adjacent focalized beams b1 and b2 do not even overlap. Accordingly, none of the received signal data related to the first beam b1 or related to the second beam b2 contains information about the non-overlapping area G. Therefore in case the focal point of the STB (not shown in fig. 1) is within the non-overlapping area G, the spatial region cannot be reconstructed at the focal point of the STB.

The disadvantage of a non-overlapping area G between the adjacent focalized beams b1 and b2 with same focal depth FD1 is thus a loss of information. Within the non-overlapping area G data about the medium cannot be gathered with this kind of setting and therefore the method becomes inefficient.

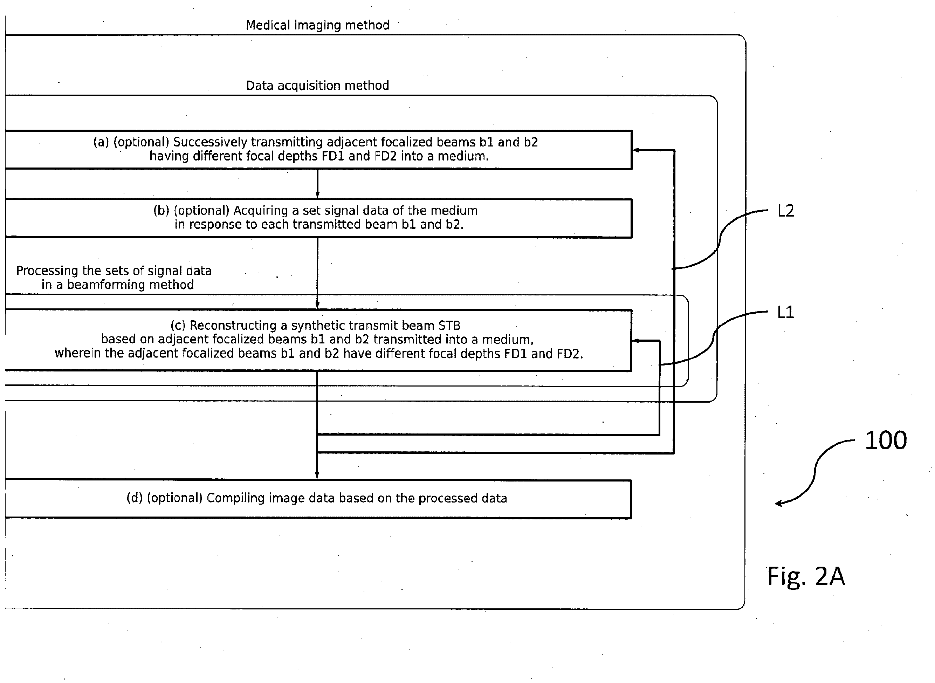

Fig. 2A shows a flowchart of a method 100 comprising reconstructing a synthetic transmit beam STB based on adjacent focalized beams transmitted into a medium according to examples of the present disclosure. The adjacent focalized beams b1 and b2 have different focal depths FD1 and FD2. The synthetic transmit beam STB may be focalized at a synthetic focal depth SFD.

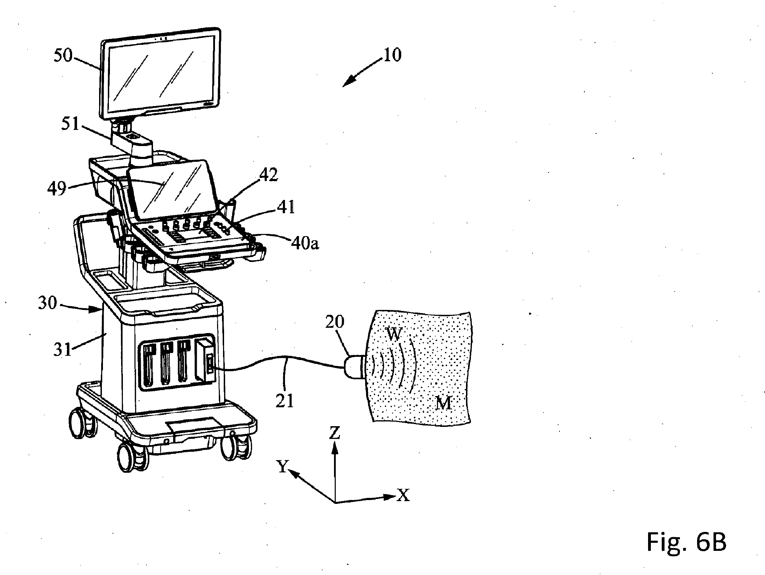

The method 100 may be carried out by means of a system, more in particular by an ultrasound system 10. An example of an ultrasound system 10 is described in context of Fig. 6B.

The method 100 may comprise a first optional operation (a) of successively transmitting adjacent focalized beams b1 and b2 having different focal depths FD1 and FD2 into a medium. In a second optional operation (b), a set signal data of the medium in response to each transmitted beam b1 and b2 may be acquired. The method 100 comprises a further operation (c) of processing the sets of signal data in a beamforming method. The beamforming method comprises reconstructing a synthetic transmit beam STB based on adjacent focalized beams b1 and b2 transmitted into a medium, wherein the adjacent focalized beams b1 and b2 have different focal depths FD1 and FD2. Additionally, the method 100 may comprise an optional operation (d) compiling image data based on the processed data.

The synthetic transmit beam STB is focalized in a spatial region of the medium M. Furthermore, a physical characteristic of the spatial region in the medium M may be determined as a function of the reconstructed synthetic transmit beam.

In other words, in a first step, the region of interest ROI might be predefined. Furthermore, the synthetic transmit beam STB may be focalized in a spatial region of the medium M, wherein the spatial region may be in the region of interest ROI. The synthetic transmit beam STB may be focalized at a synthetic focal depth SFD and in an overlapping area of the adjacent focalized beams b1 and b2.

Accordingly, a data acquisition method may comprise the operation (a) of successively transmitting adjacent focalized beams having different focal depths into a medium, the further operation (b) of acquiring a set signal data of the medium in response to each transmitted beam, and the further operation (c) of processing the sets of signal data in a beamforming method. A medical imaging method may comprise the operations of the data acquisition method and the further operation (d) compiling image data based on the processed data.

The method 100 may comprise a first loop L1. In the first loop L1, processing the sets of signal data in a beamforming method may be repeated. In other words, different synthetic transmit beams may be reconstructed in the loop L1 in respective iterations using signal data of the same transmit beams, i.e. the adjacent focalized beams b1 and b2. These STBs may have focal points located somewhere between the beams b1 and b2, i.e. in areas covered by both beams b1 and b2. Hence, image data between the two beams b1 and b2 may be reconstructed.

Regarding loop L1, it is also possible that first all sets of signal data are acquired (cf. operations a, b) and stored and then L1 is iterated for different STBs.

The method 100 may comprise a second loop L2. In the second loop L2, the data acquisition method may be repeated using different transmit beams. In other words, the data acquisition method may be repeated while different transmit beams, which may be the adjacent focalized beams b1 and b2 or other adjacent focalized beams bn and bn+1, are used in each iteration. Accordingly, in each iteration, STBs may be reconstructed having focal points located somewhere between the beams bn and bn+1, i.e. in areas covered by both beams bn and bn+1. Hence, image data between all beams bn and bn+1 may be reconstructed.

Regarding loop L2, it is also possible that first all sets of signal data are acquired (cf. operations a, b) and stored and then L2 is iterated for different STBs between different beams bn and bn+1.

In contrast, in conventional multi-focus imaging, each pixel is reconstructed based on only one beam. Accordingly, multi-focus image acquisition already leads to a relatively high amount of available data for each spatial region.

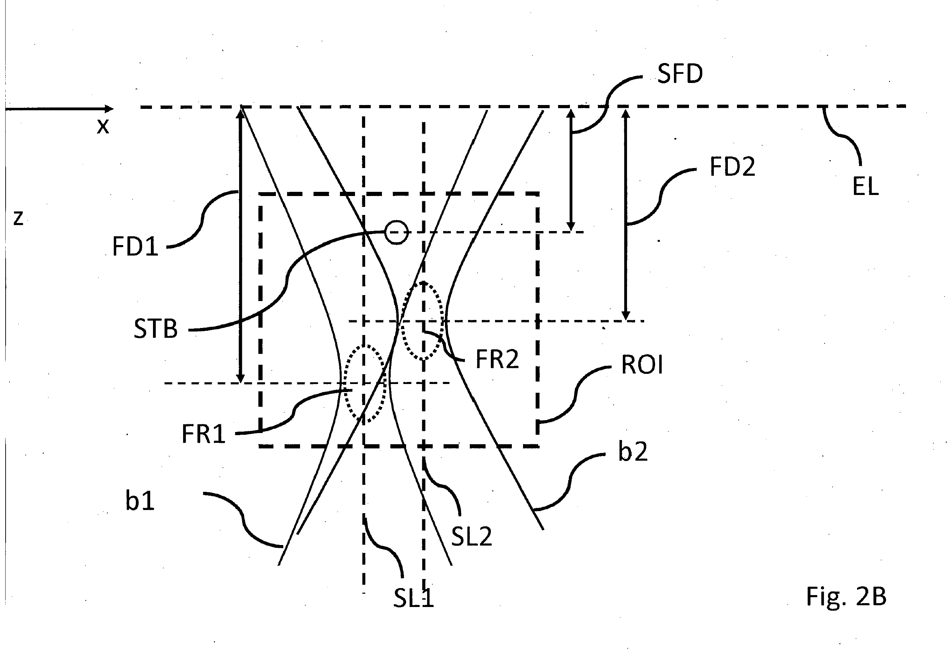

The embodiment in Fig. 2B is a further development of the example shown in Fig. 1. Fig. 2B shows two adjacent focalized beams b1 and b2 with different focal depths FD1 and FD2 according to the present disclosure, for instance according to the example of Fig. 2A. Here, focalized beam b1 has the focal depth FD1 and focalized beam b2 has the focal depth FD2. Due to the different focal depths FD1 and FD2, there is no non-overlapping area G between the two adjacent focalized beams b1 and b2. In other words, choosing adjacent focalized beams having different focal depths can prevent from obtaining a non-overlapping area G, while at the same time, keeping the same distance between the scanning line and thus maintaining the same framerate.

The relation between the focal depths FD1 and FD2 may be, for example, about 1.3.

The focalized beams b1 has a focal region FR1 more distanced from the emission line EL and the other focalized beam b2 has a focal region FR2 closer to the emission line EL. Furthermore, the adjacent focalized beams b1, b2 are arranged along adjacent scanning lines SL1, SL2.

The focal depths FD1 and FD2 may be defined as a function of a depth of a region of interest ROI in the medium. The region of interest ROI is a 3D spatial region. Here, the region of interest ROI has the shape of a cube and is shown in Fig. 2B as a square due to the section view.

Reconstructing the synthetic transmit beam STB may be conducted for a focal point of the STB, wherein the focal point of the STB is located in an overlapping area the two adjacent focalized beams b1 and b2. In other words, the synthetic transmit beam STB may be reconstructed based on signal data of the medium associated with both adjacent focalized beams b1 and b2 transmitted into the medium. Accordingly, since information (i.e. received signal data) related to the first beam b1 and information related to the second beam b2 are used to reconstruct the STB, the spatial region may advantageously be reconstructed more precisely at the focal point of the STB.

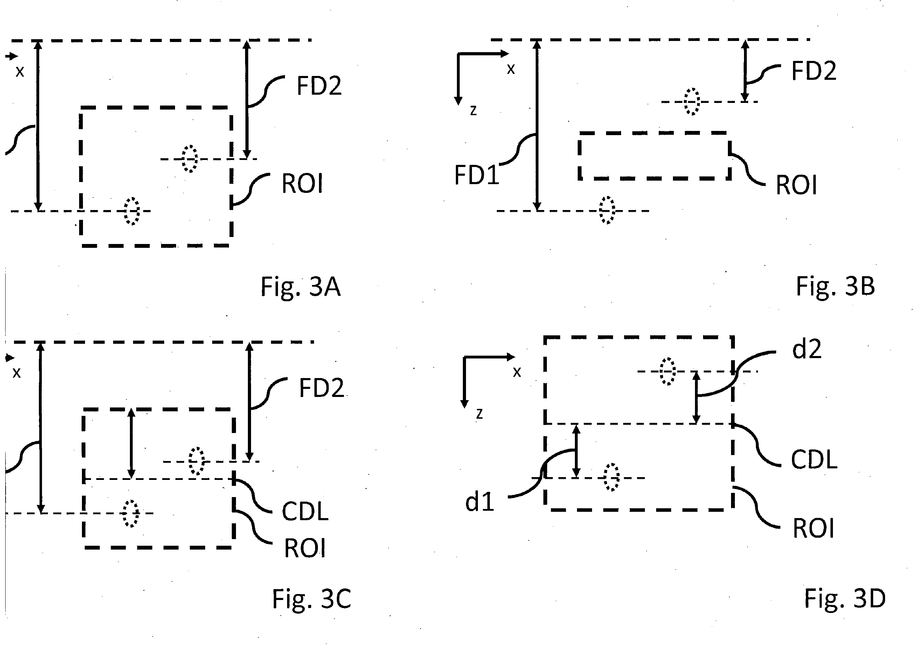

Fig. 3A shows the focal depths FD1 and FD2 within an extension of the region of interest ROI in the depth direction, according to examples of the present disclosure. In other words, the focal depths FD1 and FD2 are set such that they are both inside of the area that is defined by the region of interest ROI. The depth direction is the z direction in this illustrative example.

Fig. 3B shows the focal depths FD1 and FD2 beyond an extension of the region of interest in the depth direction according to examples of the present disclosure. In this example, the focal depths FD1 is below the region of interest ROI in depth direction and FD2 is above the region of interest ROI in depth direction.

Fig. 3C shows a first one of the focal depths above a central depth level CDL of the region of interest ROI and wherein a second one of the focal depths is below the central depth level CDL of the region of interest ROI according to examples of the present disclosure. The central depth level CDL may be a line dividing the region of interest ROI into an upper part (i.e. less deep part in the medium) and a lower part (i.e. deeper part in the medium). The upper part and the lower part of the region of interest ROI may have the same size. Here, the focal depth FD2 is above the central depth level CDL of the region of interest ROI and the focal depth FD1 is below the central depth level CDL of the region of interest ROI.

Fig. 3D shows the first focal depth FD1 and the second focal depth FD2 having the same distance from the central depth level CDL of the region of interest ROI, according to examples of the present disclosure. Here, the first focal depth FD1 has a first distance d1 between the central depth level CDL and the focal point of the first focal depth FD1. The second focal depth FD2 has a second distance d2 between the central depth level CDL and the focal point of the second focal depth FD2. The first distance d1 and the second distance d2 may be similar or equal (d1=d2) in this example.

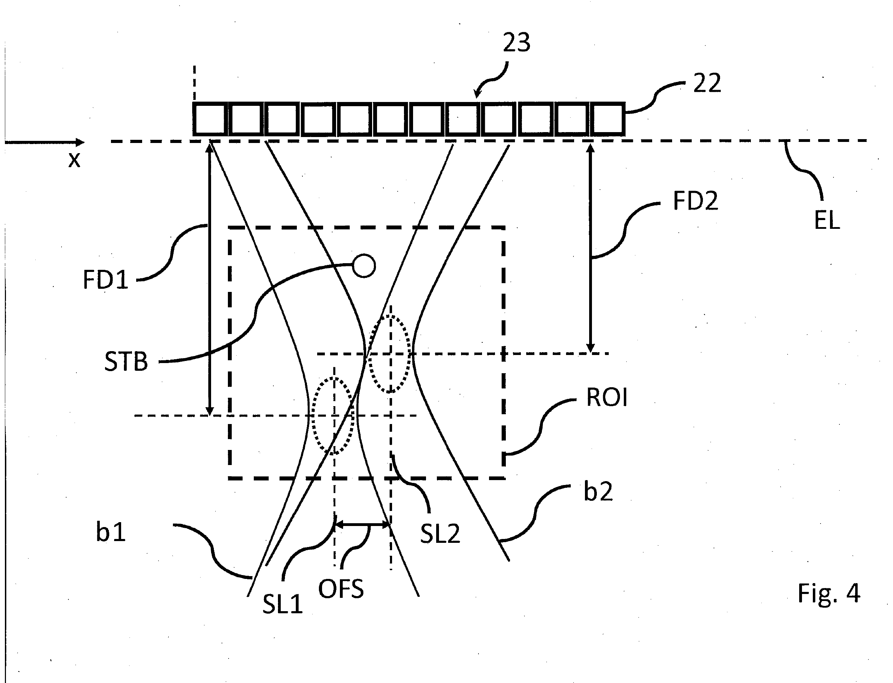

Fig. 4 shows the adjacent focalized beams b1 and b2 that are offset OFS to each other and the adjacent focalized beams b1 and b2 are transmitted by a plurality of transducer elements 22 into the medium, according to examples of the present disclosure. A transducer array 23 may comprise the plurality of transducer elements 22 and the transducer elements 22 may be arranged along scanning lines SL1, SL2.

The offset OFS between the scanning lines SL1, SL2 is in a direction perpendicular to a depth direction and a primary axis of the beams. The offset OFS is a shift in x direction.

Accordingly, the adjacent focalized beams are adjacent in a direction perpendicular to the depth direction or z direction. In other words, the adjacent focalized beams are adjacent to each other in a lateral dimension (i.e. the x dimension in fig. 4) of the medium M.

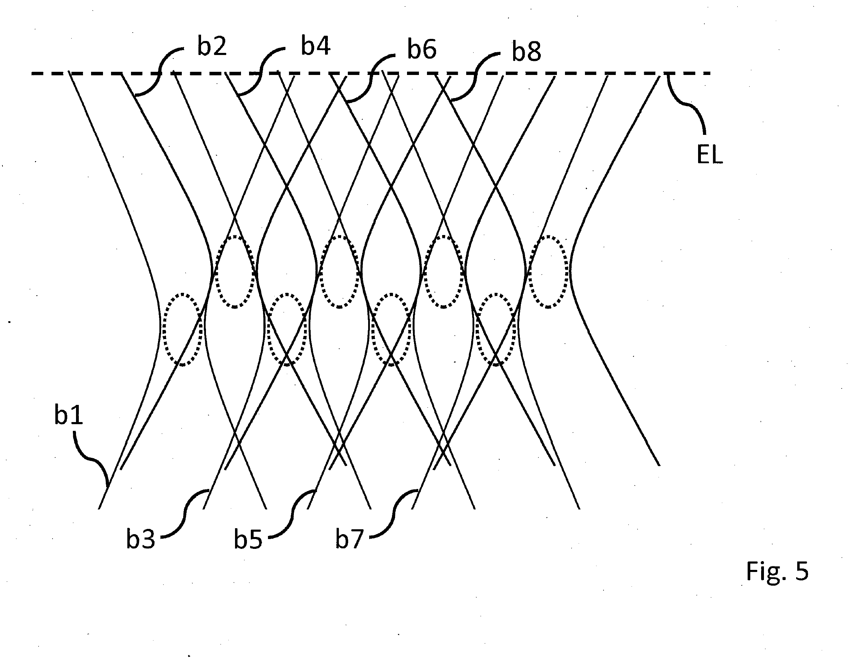

In fig. 5 more than two adjacent focalized beams b1, b2, b3, b4, b5, b6, b7, and b8 are transmitted into the medium having two (or more; not shown in fig. 5) different focal depths according to examples of the present disclosure. On the one hand, the focalized beams b1, b3, b5, and b7 may have the same focal depth. On the other hand, the focalized beams b2, b4, b6, and b8 may have the same focal depth. Desirably there may be no gaps (i.e. non-overlapping areas) between the focalized beams b1, b2, b3, b4, b5, b6, b7, and b8. One advantage of this embodiment is that a big amount of data about the medium may be obtained. Another advantage is that there may be no data missing out to non-overlapping areas or gaps.

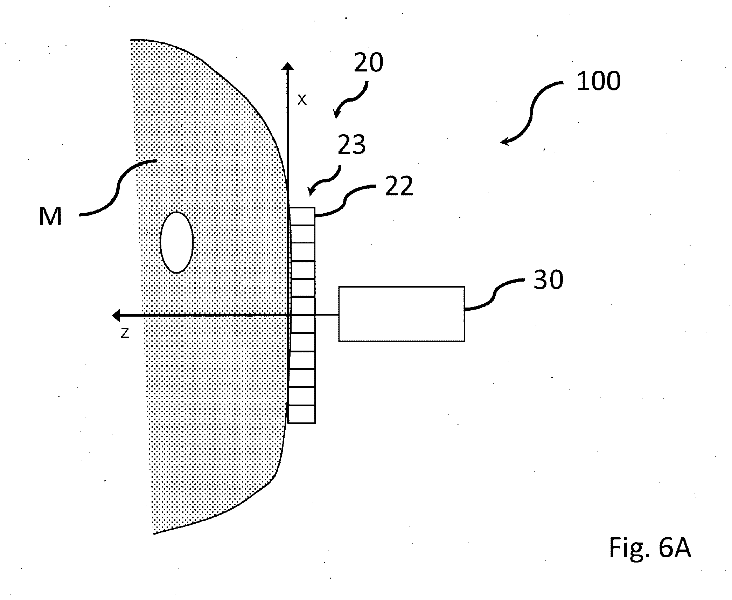

Fig. 6A shows a schematic drawing of an ultrasound system 100 according to examples of the present disclosure. The system 100 shown on Fig. 6A is adapted for collecting ultrasound spatio-temporal signal data of the medium M, for instance living tissues and/or in particular human tissues of a person. The system 100 is further configured to construct ultrasound spatio-temporal signal data of the medium M in an optimized manner by taking specular properties of the medium into account. The system may include for instance:

- A plurality of transducer elements 22. The transducer elements may be configured to transmit (a) a pulse into the medium and/or to receive (b) a plurality of signals from the medium, optionally in response to transmitting (a) the pulse into the medium. A transducer array comprising a plurality of transducer elements 22 may be used. For example, a linear array 23 may be provided, typically including a few tens of transducer elements (for instance 128, 256, or more) juxtaposed along an axis X (horizontal or array direction X). In this example, the array 23 is adapted to perform a bidimensional (2D) imaging of the medium M, but the array 23 could also be a bidimensional array adapted to perform a 3D imaging of the medium M. Accordingly, a matrix of transducers may be used. It is though also possible that the system comprises a single line of transducers moveable in a probe, such that 3D imaging can be achieved. The transducer array 23 may also be a convex array including a plurality of transducer elements aligned along a curved line. The same transducer element(s) may be used to transmit a pulse and receive the response, or different transducer elements may be used for transmission and reception. There may be one or more emitting transducer elements and a plurality of receiving transducer elements. In a further alternative, only a single transducer element 22 may be used, receiving a plurality of signals which have different spatial properties (for example originating from different spatial regions). The transducer element 22 may for instance be electronically or physically moveable;
- an electronic control device 30, controlling the transducer array and acquiring signals therefrom. The electronic control device 30 may be part of a probe 20 which also comprises the transducer elements 22. Alternatively, the electronic control device 30 may be external to the probe 20, or consist of several devices which are partially part of the probe 20 and partially external thereto.

The system may further include a processing unit (not shown) for controlling the electronic control device 30 and/or for example for sending data to an external device, such as for example, a server, a computer on which an artificial intelligence (AI) algorithm is running, a dedicated workstation, presenting data, a device for displaying images obtained from the electronic control device or any of the other external devices. Accordingly, the method according to the present disclosure, in particular a beamforming method, may be carried out by at least one of the electronic control devices 30, the processing unit or any of the external devices. Furthermore, the process for compiling the ultrasound image data may be carried out by the same processing device as that one for optimizing the optimizing a process, or (at least in part) by another one.

According to further examples, the system 100 may include at least one processing unit (or processor) and memory. In examples, the processor and memory unit may be incorporated into the system such as depicted in FIG. 6A or may be a computer or computer communicatively linked thereto. Depending on the exact configuration and type of computing device, memory (storing, instructions to evaluate ultrasound data or otherwise perform the methods described herein) may be volatile (such as RAM), non-volatile (such as RAM, flash memory, etc.), or some combination of the two. Further, the system 100 may also include storage devices (removable and/or non-removable) including, but not limited to, magnetic or optical disks or tape. Similarly, the system 100 may also have input device(s) such as keyboard, mouse, pen, voice input, etc. and/or output device(s) such as a display, speakers, printer, etc. Also included in the environment may be one or more communication connections, such as LAN, WAN, point to point, etc. In embodiments, the connections may be operable to facility point-to-point communications, connection-oriented communications, connectionless communications, etc.

The system 100 may typically include some form of computer readable media. Computer readable media can be any available media that can be accessed by processing unit (or processor) or other devices comprising the operating environment. By way of example, and not limitation, computer readable media may comprise computer storage media and communication media. Computer storage media includes volatile and nonvolatile, removable and non-removable media implemented in any method or technology for storage of information such as computer readable instructions, data structures, program modules or other data. Computer storage media does not include communication media.

Communication media embodies computer readable instructions, data structures, program modules, or other data in a modulated data signal such as a carrier wave or other transport mechanism and includes any information delivery media. The term "modulated data signal" means a signal that has one or more of its characteristics set or changed in such a manner as to encode information in the signal. By way of example, and not limitation, communication media includes wired media such as a wired network or direct-wired connection, and wireless media such as acoustic, RF, infrared, microwave, and other wireless media. Combinations of the any of the above should also be included within the scope of computer readable media.

The system 100 may be a single computer operating in a networked environment using logical connections to one or more remote computers. The remote computer may be a personal computer, a server, a router, a network PC, a peer device or other common network node, and typically includes many or all of the elements described above as well as others not so mentioned. The logical connections may include any method supported by available communications media:

The transducer elements 22 may comprise piezo-crystals and/or other components that may be configured to generate and/or record and/or receive signals. The terms transducer and transducer elements may be used synonymously throughout this disclosure unless denoted differently.

Transducer elements 22 may be configured to generate and/or record and/or receive signals, optionally ultrasonic signals. Transducer elements 22 and/or electronic control device 30 and/or the processing unit may be configured to determine phase properties of spatio-temporal signal data.

The axis z on Fig. 6A is an axis perpendicular to the axis x, for example in the depth direction of the examined medium M. This direction is designated in the present document as a vertical or axial direction. An ultrasound beam emitted in the direction of the z-axis may be understood as having a tilt angle of 0°.

Fig. 6B shows a schematic drawing of an ultrasound imaging system 10 according to examples of the present disclosure. The ultrasound imaging system 10 may be an example of a medical imaging system 200, as described in context of Fig. 6A.

The ultrasound imaging system 10 may comprise:

- a probe 20 (for example corresponding to the probe 20 of fig. 6A),
- a processing unit 30 for processing an image on the bases of signals received by the probe,
- a control panel 40 connected to the processing unit, said control panel at least comprising buttons 41 and a touch pad 42, and
- a display 50 for visualizing the image.

The probe 20 may be connected to the processing unit 30 via a cable 21 or via a wireless connection, and it is able to emit ultrasound waves W into a medium M and to receive ultrasound waves W from the medium M, said received ultrasound waves being consequent or resulting from reflections of said emitted ultrasound waves on diffusing particles inside said medium M. The probe 20 may comprise a transducer array 23 comprising a plurality of transducer elements 22, each one converting an electric signal into a vibration and reciprocally. A transducer element 22 may be, for example, a piezoelectric element. The transducer array 23 may comprise hundred transducers or more. The transducer array 23 is a linear or curved and is disposed on an outer surface of the medium M so as to be coupled to the medium M and to vibrate and to emit or receive ultrasound waves W.

The display screen 50 may be a screen for visualizing the image processed by the processing unit 30. The display 50 may also visualize other information such as scales used in the image, or configuration information for the processing or any information such as help information or contextual gesture help for the touch pad 42.

The display screen may by articulated on a support arm 51 for better positioning for the user. The display screen is usually a high definition screen of a great size (at least 20 inches) for better image visualization to the user.

The control panel 40a is for example a portion of the system casing 31, said portion comprising a panel casing having a substantially flat surface inclined towards the user for manipulation by one hand of said user. The control panel 40a may be moved by a hanger upwards and downward for being adapted to the user size, and may be optionally moved frontward and rearward for being adapted to the user position. As seen on Fig. 6B, the control panel 40a may include a control panel display screen 49 for visualizing several configuration information or any information dedicated to the user . This control panel display screen 49 can also be hinged to the control panel casing 48 for being inclined into a different inclination than the control panel surface.

Throughout the description, including the claims, the term "comprising a" should be understood as being synonymous with "comprising at least one" unless otherwise stated. In addition, any range set forth in the description, including the claims should be understood as including its end value(s) unless otherwise stated. Specific values for described elements should be understood to be within accepted manufacturing or industry tolerances known to one of skill in the art, and any use of the terms "substantially" and/or "approximately" and/or "generally" should be understood to mean falling within such accepted tolerances.

The terms "record" and "receive" may be used synonymously throughout this disclosure unless denoted differently.

Although the present disclosure herein has been described with reference to particular examples, it is to be understood that these examples are merely illustrative of the principles and applications of the present disclosure.

It is intended that the specification and examples be considered as exemplary only, with a true scope of the disclosure being indicated by the following claims.

A reference herein to a patent document or any other matter identified as prior art, is not to be taken as an admission that the document or other matter was known or that the information it contains was part of the common general knowledge as at the priority date of any of the claims.

**Claims**

1. A beamforming method (100),
   comprising:

   > reconstructing a synthetic transmit beam (STB) based on adjacent focalized beams (b1, b2) transmitted into a medium (M), wherein
   > the adjacent focalized beams (b1, b2) have different focal depths (FD1, FD2).

2. The method according to claim 1, wherein
   the focal depths (FD1, FD2) are defined as a function of a depth of a region of interest (ROI) in the medium (M).

3. The method according to the preceding claim, wherein
   the focal depths (FD1, FD2) are defined to be within or beyond an extension of the region of interest (ROI) in the depth direction.

4. The method according to the preceding claim, wherein
   a first one of the focal depths (FD1) is above a central depth level (CDL) of the region of interest (ROI) and wherein a second one of the focal depths (FD2) is below the central depth level (CDL) of the region of interest (ROI).

5. The method according to the preceding claim, wherein
   the first focal depth (FD1) and the second focal depth (FD2) have the same distance (d1, d2) from the central depth level (CDL) of the region of interest (ROI).

6. The method according to any one of the preceding claims, wherein

   > the transmitted beams (b1, b2) have different apertures, and/or
   > an aperture of a transmitted beam is determined as a function of its focal depth (FD1, FD2).

7. The method according to any one of the preceding claims, wherein
   more than two adjacent focalized beams (b1, b2, b3, b4, b5, b6, b7, b8) are transmitted into the medium having two or more different focal depths.

8. The method according to the preceding claim, wherein
   the different focal depths of the more than two adjacent focalized beams (b1, b2, b3, b4, b5, b6, b7, b8) are alternating.

9. The method according to any one of the preceding claims, wherein

   > the focal depths (FD1, FD2) are selected such that the adjacent focalized beams (b1, b2) overlap in the region of interest (ROI) in the medium (M)
   > and/or an overlap between the adjacent focalized beams (b1, b2) in a region of interest (ROI) of the medium (M) is increased.

10. The method according to any one of the preceding claims, wherein

    > the adjacent focalized beams (b1, b2) are offset to each other, and/or
    > the adjacent focalized beams (b1, b2) are arranged along adjacent scanning lines (SL1, SL2).

11. The method according to any one of the preceding claims, wherein

    > the adjacent focalized beams (b1, b2) are based on ultrasound waves transmitted into the medium (M), and/or the adjacent focalized beams (b1, b2) correspond to physical areas of the medium (M) passed through by ultrasound waves.

12. The method according to any one of the preceding claims, wherein
    reconstructing the synthetic transmit beam (STB) comprises:

    > reconstructing the synthetic transmit beam (STB) based on signal data of the medium (M) associated with the adjacent focalized beams (b1, b2) transmitted into the medium (M).

13. The method according to any one of the preceding claims, wherein
the signal data comprise at least one of in-phase and quadrature phase, IQ, data, wherein a phase of the IQ data is adjusted as a function of a synthetic focal depth (SFD) of the synthetic transmit beam (STB) in relation to the focal depths (FD1, FD2) of the adjacent focalized beams (b1, b2).

14. The method according to any one of the preceding claims,
further comprising:

determining a physical characteristic of a spatial region in the medium (M) as a function of the reconstructed synthetic transmit beam (STB), wherein the synthetic transmit beam (STB) is focalized in the spatial region.

15. A data acquisition method, comprising:

successively transmitting adjacent focalized beams (b1, b2) having different focal depths (FD1, FD2) into a medium (M),
acquiring a set signal data of the medium (M) in response to each transmitted beam,
processing the sets of signal data in a beamforming method according to any one of the preceding claims.

16. A medical imaging method, comprising:
the data acquisition method according to the preceding claim, and compiling image data based on the processed data.

17. A computer program comprising computer-readable instructions which when
executed by a data processing unit cause the data processing unit to carry out processing operations of the method according to any one of preceding claims.

18. A beamforming system,
the system comprising a processing unit (30) configured to:

reconstruct a synthetic transmit beam (STB) based on adjacent focalized beams (b1, b2) transmitted into a medium (M), wherein
the adjacent focalized beams (b1, b2) have different focal depths (FD1, FD2).

Prior art
Fig. 1

Medical imaging method

Data acquisition method

(a) (optional) Successively transmitting adjacent focalized beams b1 and b2
having different focal depths FD1 and FD2 into a medium.

(b) (optional) Acquiring a set signal data of the medium
in response to each transmitted beam b1 and b2.

Processing the sets of signal data
in a beamforming method

(c) Reconstructing a synthetic transmit beam STB
based on adjacent focalized beams b1 and b2 transmitted into a medium,
wherein the adjacent focalized beams b1 and b2 have different focal depths FD1 and FD2.

(d) (optional) Compiling image data based on the processed data

L2

L1

100

Fig. 2A

Fig. 2B

Fig. 3A

Fig. 3B

Fig. 3C

Fig. 3D

Fig. 4

EP 4 394 441 A1

Fig. 5

Fig. 6A

EP 4 394 441 A1

EP 4 394 441 A1

Fig. 6B

Europäisches
Patentamt

European
Patent Office

Office européen
des brevets

# EUROPEAN SEARCH REPORT

Application Number

EP 22 31 5360

## DOCUMENTS CONSIDERED TO BE RELEVANT

| Category | Citation of document with indication, where appropriate, of relevant passages | Relevant to claim | CLASSIFICATION OF THE APPLICATION  (IPC) |
|---|---|---|---|
| X | EP 3 207 878 A1 (HITACHI LTD [JP]) 23 August 2017 (2017-08-23) * abstract; figures 2, 4 - 7, 9, 10 * * paragraphs [0001], [0015] - [0017] * * paragraph [0021] - paragraph [0026] * * paragraph [0028] - paragraph [0034] * ----- | 1-18 | INV. G01S7/52 G01S15/89 ADD. A61B8/08 G10K11/34 |

TECHNICAL FIELDS
SEARCHED      (IPC)

G01S
A61B
G10K

The present search report has been drawn up for all claims

| Place of search | Date of completion of the search | Examiner |
|---|---|---|
| Munich | 26 May 2023 | Knoll, Bernhard |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

...............................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 31 5360

This annex lists the patent family members relating to the patent documents cited in the above-mentioned European search report.
The members are as contained in the European Patent Office EDP file on
The European Patent Office is in no way liable for these particulars which are merely given for the purpose of information.

26-05-2023

| Patent document cited in search report | | Publication date | Patent family member(s) | | Publication date |
|---|---|---|---|---|---|
| EP 3207878 | A1 | 23-08-2017 | CN | 107072641 A | 18-08-2017 |
| | | | EP | 3207878 A1 | 23-08-2017 |
| | | | JP | 6014643 B2 | 25-10-2016 |
| | | | JP | 2016077442 A | 16-05-2016 |
| | | | US | 2017238908 A1 | 24-08-2017 |
| | | | WO | 2016060017 A1 | 21-04-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20220287685 A1

- WO 2017093778 A

**Non-patent literature cited in the description**

- **MOO-HO BAE ; MOK-KUN JEONG.** A study of synthetic-aperture imaging with virtual source elements in B-mode ultrasound imaging systems. *IEEE Transactions on Ultrasonics, Ferroelectrics, and Frequency Control,* November 2000, vol. 47 (6), 1510-1519

- **T. HERGUM ; T. BJASTAD ; K. KRISTOFFERSEN ; H. TORP.** Parallel beamforming using synthetic transmit beams. *IEEE Transactions on Ultrasonics, Ferroelectrics, and Frequency Control,* February 2007, vol. 54 (2), 271-280